# EUROPEAN PATENT SPECIFICATION

(11) **EP 1 076 561 B1**
(45) Date of publication and mention of the grant of the patent: **30.03.2005**
(21) Application number: 99921972.8
(22) Date of filing: 14.05.1999
(51) Int. Cl.: A61K 31/505, A61K 39/395, A61K 31/385, A61K 39/39, A61P 35/00

(54) **COMBINATION THERAPY FOR THE TREATMENT OF TUMORS**
KOMBINATIONSTHERAPIE ZUR BEHANDLUNG VON TUMOREN
THERAPIE COMBINEE POUR LE TRAITEMENT DE TUMEURS

(30) Priority: 15.05.1998 US 85687 P
(43) Date of publication of application: 21.02.2001
(73) Proprietor: APHTON CORPORATION, Woodland, CA 95776-1049 (US)
(72) Inventor: GEVAS, Philip, C., Key Biscayne, FL 33149 (US); GRIMES, Stephen, Davis, CA 95616 (US); KARR, Stephen, L., Davis, CA 95616 (US); WATSON, Susan, A., Nottingham NG2 6RB (GB); MICHAELI, Dov, Larkspur, CA 94939 (US)
(74) Representative: Lucas, Brian Ronald
(86) International application number: PCT/US1999/010750
(87) International publication number: WO 1999/059628

(56) References cited:
- EP-A- 0 380 230
- EP-A- 0 755 683
- AJANI J A ET AL: "PHASE I AND II STUDIES OF THE COMBINATION OF RECOMBINANT HUMAN INTERFERON- AND 5-FLUOROURACIL IN PATIENTS WITH ADVANCED COLORECTAL CARCINOMA" JOURNAL OF BIOLOGICAL RESPONSE MODIFIERS,US,RAVEN PRESS. NEW YORK, vol. 8, no. 2, page 140-146 XP000032195
- WATSON S A ET AL: "ANTI-GASTRIN ANTIBODIES RAISED BY GASTRIMMUNE INHIBIT GROWTH OF THEHUMAN COLORECTAL TUMOUR AP5" INTERNATIONAL JOURNAL OF CANCER,US,NEW YORK, NY, vol. 61, no. 2, page 233-240 XP000654518 ISSN: 0020-7136
- WATSON S. A. ET AL.,: "A comparison of an anti-gastrin antibody and cytotoxic drugs in the therapy of human gastric ascites in scid mice" INT. J. CANCER, vol. 81, - 12 April 1999 (1999-04-12) pages 248-254, XP000853851

### SEQUENCE LISTING

<110> APHTON CORPORATION
<120> Combination Therapy for the Treatment of Tumors
<130> 1102865-0034
<140> US
   <141> 1999-05-14
<150> US 60/085,687
   <151> 1998-05-15
<160> 2
<170> PatentIn Ver. 2.0
<210> 1
   <211> 9
   <212> PRT
   <213> human or synthetic peptide
<220>
   <221> MOD_RES
   <222> (1)
   <223> pyroglutamic acid
<400> 1
   Glu Gly Pro Trp Leu Glu Glu Glu Glu
    1               5
<210> 2
   <211> 7
   <212> PRT
   <213> Artificial Sequence
<220>
   <223> Description of Artificial Sequence:Synthetic peptide
<400> 2
   Ser Ser Pro Pro Pro Pro Cys
    1               5

## Description

### FIELD OF INVENTION

The invention is related to a tumor therapy for inhibiting growth by neutralizing immunologically the growth stimulating peptide hormones in combination with a chemotherapy applying a 5-fluorouracil derivative and leucovorin.

### BACKGROUND OF INVENTION

Gastrin is a peptide hormone which occurs in two mature forms, tetratriacontagastrin (G34) and heptadecagastrin (G17), and is synthesized and secreted by specialized cells, G cells, that are located in the stomach antrum. In gastrin-producing cells, these gastrin hormones are posttranslationally processed from a common precursor molecule termed "preprogastrin" containing a signal peptide. The signal peptide "pre" is removed in the endoplasmic reticulum of the cell, resulting in the "progastrin" peptide, which is in turn further processed in the cell to yield the mature gastrins G34 and G17, before secretion into the bloodstream (Dickinson 1991). (The full citations for the references cited herein are provided in the Reference Section preceding the Claims). Both mature forms of G34 and G17 are amidated at their carboxy-terminal end (-NH₂). In humans, multiple forms of G17 have been found resulting from differential processing of the precursor molecule, each of which may have different biological activities (Dickinson 1995 and Ciccotosto et al. 1995). In the posttranslational processing of gastrin, it is the "mature" carboxy-amidated form that binds to a specific cell receptor, the so-called CCK-B/gastrin receptor, via the carboxy terminus of the peptide (Kopin et al. 1992).

The gastrin hormones are secreted into the circulating blood and bind to specific cells in the stomach, namely, enterochromaffin-like (ECL) cells and parietal cells, that indirectly or directly affect stomach acid output. Historically, both gastrin hormones have been associated with the stimulation of gastric acid secretion (Edkins, J.S. 1905). In recent years, evidence has accumulated showing that gastrin also acts as a trophic factor within the gastrointestinal tract (Johnson, L. 1992) and that it promotes the growth of gastrointestinal cancers (Watson et al. 1989, Dickinson, C.J. 1995), as well as nongastrointestinal cancers, including small cell carcinoma of the lung (Rehfeld et al. 1989).

Several types of tumors, including colorectal, stomach, pancreatic and hepatocellular adenocarcinomas possess CCK-B/gastrin receptors in their plasma membranes and the tumor cells respond to gastrin with powerful cellular proliferation (Rehfeld, J.F. 1972, Upp et al. 1989 and Watson et al. 1993). Elevated plasma levels of total gastrin occur in patients with colorectal cancers. and. in particular. increased amounts of the hormone precursor progastrin have been detected in many colorectal tumors using gastrin antisera (Ciccotosto et al. 1995). More recently,it has been discovered that many of these cancer cells also secrete gastrin and thus effect an autonomous proliferative pathway(Van-Solinge et al. 1993 ,Nemeth et al. 1993 and Seva et al. 1994).

The peptide hormones G17 and G34 bind to the CCK-B/gastrin receptors on the cell membranes of normal cells.However, it has been found that G17,but not G34, stimulates the growth of gastrin-dependent cancer cells. Serum associated G17, in particular, has the potential to stimulate the growth of colorectal tumors in an endocrine manner mediated by CCK-B/gastrin receptors in tumor cells(Watson et al. 1993).G17 is particularly implicated in stimulating the growth of colorectal adenocarcinomas due to a possible increased affinity for the CCK-B/gastrin receptors on the tumor cells, as compared to other gastrin hormone species (Rehfeld 1972 and Van Solinge et al., 1993). The CCK-B/gastrin receptors were found to be expressed in a high affinity form on 56.7% of human primary colorectal tumors (Upp et al. 1989).

Numerous studies have shown that, in addition to being able to respond to exogenous endocrine gastrin, human gastric and colorectal tumors produce gastrin and its precursors (Ciccotosto et al., 1995; Finley et al., 1993; Kochman et al., 1992; Nemeth et al., 1993; Van Solinge et al., 1993), thus effecting an autocrine growth stimulatory pathway. Gastrin production in tumor cells differs from that of endocrine G cells. Specifically, those tumor cells contain a high proportion of the precursor progastrin along with a lower concentration of mature peptides.This abnormal ratio is postulated to be due to constitutive unregulated release of gastrin combined with a limited activity of peptidylglycine α-amidating monooxygenase (Ciccotosto et al.. 1995: Kelly, 1998). Thus, the unregulated release of gastrin leads to the abnormal production and secretion of different molecular forms of the hormone.Specifically,colon carcinoma cells do not efficiently process progastrin resulting in less conversion of precursor gastrin to the mature peptides and, thus produce mostly incomplete or aberrant gastrins, (Dickinson 1995 and Rehfeld et al. 1993). In addition, the increased gastrin level in colorectal tumors is, in part, attributed to the aberrant expression of the gastrin gene in the colorectal tumor cells(Hoosein et al. 1990, Baldwin et al. 1992 and Finley et al. 1993). Gastrin-like peptides have been identified in such cells (Hoosein et al. 1988,Watson et al. 1991a and Finley et al.1993), and were confirmed to be precursor gastrin species (Van-Solinge et al. 1993 and Nemeth et al. 1993).

The presence of amidated-G17 (G17-NH₂) in some colorectal cancers (Ciccotosto et al., 1995; Van Solinge et al., 1993) demonstrates that some tumors retain an intact processing pathway. as gastrin amidation only occurs in secretory granules (Varro et al., 1994). Endogenously produced gastrin also acts as an autocrine growth factor, since the basal growth of a colorectal cell line was shown to be inhibited by an anti-gastrin antibody (Hoosein et al., 1988). This was confirmed in a second study in which Northern blot analysis revealed gastrin mRNA in the same cell lines and radioimmunoassay revealed gastrin-like immunoreactivity in cell culture supernatant (Hoosein et al., 1990). Gastrin peptides also possess paracrine roles (Watson et al., 1991b)which was confirmed(Finley et al., 1993)in experiments showing gastrin immunoreactivity more predominant in subpopulations of malignant colorectal mucosal cells.

When G 17 binds to its receptor a G 17/receptor complex is formed which stimulates cell growth by way of secondary messengers for regulating cell function (Yamada et al. 1993).

The binding of G17 to the CCK- B/gastrin receptor leads to activation of phosphatidylinositol breakdown, the protein kinase C activation with a resultant increase in intracellular calcium ion concentration, and the induction of *c-fos* and *c-jun* protooncogenes via the mitogen-activated protein kinase, which has been implicated in the regulation of cell proliferation (Todisco et al. 1995). Additionally, gastrin binding to the CCK-B/gastrin receptor has been associated with the subsequent increase in phosphorylation by a tyrosine kinase, the pp 125FADK(focal adhesion kinase), which may also have a role in the transmission of mitogenic signals (Taniguchi et al. 1994).

Colorectal cancer remains a formidable disease to treat, as only minor improvements in survival have been obtained in recent years.Surgery is an effective treatment of the primary disease, but it is ineffectual against residual occult disease,which is frequently present. Radiation therapy post-surgery is generally recommended for patients with rectal cancers to reduce the risks of recurrence of the disease. Chemotherapy with 5-fluorouracil(5-FU) has been the most traditional effective therapy following surgery in patients with more advanced colorectal cancers.However, 5-FU therapy has been shown to be only of marginal benefit to the patient,since 5-FU is highly toxic and the therapy is costly and does not appear, alone or in combination with other cytotoxic drugs, to significantly prolong survival. In most instances, occult or inoperable colorectal tumors do not respond well to chemotherapy or radiation, and new treatments are needed to supplement present procedures.

Recently, several studies have shown that adjuvant combination chemotherapy with 5-FU and Leucovorin improves the efficacy of 5-FU in patients with advanced colorectal cancer. Leucovorin is a folic acid derivative, also known as folinic acid, Citrovorum factor, or 5-formyl-5,6,7,8,-tetrahydrofolic acid. The studies show that in Dukes Stage C patients. 5-FU/Leucovorin combination therapy may reduce mortality by 10 to 15% (Moertel. 1994). In the same patient group, combined intravenous and intraperitoneal therapy with 5-FU/leucovorin resulted in a non-significant trend to disease-free survival and overall survival advantage (Scheithauer et al., 1995). In advanced disease, the same drug combination may give rise to a survival advantage (Taylor, 1993), which has been shown to be 13.5 months of median survival in the combination group compared to 7.5 months in 5-FU-treated patients (Petrioli et al., 1995). However, this combination chemotherapy is not without significant morbidity and causes deleterious side effects including, stomatitis, diarrhea and myelosuppression (Mahood et al., 1991: Erlichman et al., 1988; Pietnelli et al., 1989). making quality of life an issue, especially in patients with advanced disease.

A number of high affinity CCK-B/gastrin receptor antagonists have been evaluated therapeutically both *in vitro* and *in vivo* in a number of experimental gastrointestinal cancers. For example, proglumide, a glutamic acid derivative (Seva et al. 190: Harrison et al. 1990 and Watson et al. 1991 a): Benzotript. an N-acyl derivative of tryptophan; L-365.260, a derivative of Aspercillin (Bock et al. 1989); and CI-988, a molecule that mimics the C-terminal pentapeptide sequence of CCK (Hughes et al. 1990), have been shown to effectively neutralize the effects of exogenous gastrin on gastrointestinal tumor growth both *in vitro* and *in vivo* (Watson et al. 1991b and Romani et al. 1994). However, these antagonists have severe toxic side effects and lack specificity, as they block the action of all potential ligands of the receptor such as G34 and CCK in normal cells. Recently, highly potent and selective CCK-B/gastrin receptor antagonists such as YM022 (Yuki et al., 1997) and YF476 (Takinami et al., 1997) have been also described.

Proglumide and Benzotript have been widely assessed in preclinical studies. The main problem with these compounds is their lack of potency, with relatively high concentrations required to displace G17 (Watson et al., 1992a; Watson et al., 1992b). Despite this, proglumide and Benzotript inhibited the basal and gastrin-stimulated proliferation of a number of cell lines (Seva et al., 1990; Watson et al., 1991a). In addition, proglumide increased the survival of xenograft mice bearing the gastrin-sensitive mouse colon tumor MC26 to 39 days in the treated animals from 25 days in the control animals.

Due to the low specificity of this class of gastrin antagonizing agents for the gastrin/CCK-B receptor, the inhibition of growth is also thought to be induced by a gastrin-receptor-independent action. Moreover, the cellular receptors which recognize and bind the gastrin do not bind all the inhibitors tested (Seva et al. 1994). Thus, if complete inhibition of gastrin binding to the receptor does not occur in the autocrine growth cascade, the gastrin antagonists may be unable to block this mechanism of tumor growth promotion.

Thus, novel therapeutic approaches are needed both as modalities in their own right and for combination strategies with chemotherapy. Combined treatments offer the possibilities of enhancing the therapeutic index and/or reducing the dose of chemotherapy required, thereby limiting the disadvantageous side effects.

A therapeutic method of selectively immunologically neutralizing the biological activity of the gastrin hormone would provide an effective means of controlling or preventing the pathologic changes resulting from excessive gastrin hormone production associated with colorectal cancers.

Coassigned U.S. Patents Nos. 5,023,077; 5,468.494; 5.607,676; 5,609,870 and 5,622.702 disclose immunogens and immunogenic compositions useful for controlling G17 and G34 levets in a patient by generating anti-gastrin antibodies and also disclose the use of such compositions for the treatment of gastric and duodenal ulcers and gastrin-induced cancers. The present invention concerns the use of the anti-G17 immunogens and immunogenic compositions disclosed in Patent Nos. 5,023,077; 5,468,494; 5,607,676; 5,609,870 and 5,662,702 in a combination therapy with chemotherapeutic agents for treating gastrin-dependent colorectal cancers.

The method of cancer therapy described herein has several advantages over present colorectal cancer treatment methods. The anti-G17 immunization. in combination with chemotherapeutic agents such as 5-FU and Leucovorin, increases the therapeutic effects in controlling or inhibiting colorectal tumor growth over chemotherapy alone.

### SUMMARY OF THE INVENTION

The present invention provides a combination therapy for treating tumors comprising immunologically neutralizing peptide hormones and factors which promote tumor cell division in combination with chemotherapy. In particular, the present invention provides a method for treating gastrin-dependent cancers, such as colorectal adenocarcinomas. The method comprises a combination therapy comprising anti-G17 immunization of the patient in need of the therapy, in conjunction with the administration of one or more chemotherapeutic agents. The anti-G17 immunization for treating gastrin-dependent tumors is surprisingly effective in generating anti-G17 antibodies, despite the known myelo-suppressive effects of the chemotherapeutic agents used.

The anti-G17 immunization comprises the active or passive immunization of a patient with an anti-G17 immunogen against the hormone G17 in order to control the patient's G17 levels. As the result of induction of anti-G17 antibodies in a patient, the G17 hormone is neutralized *in vivo* and its physiological effects are inhibited, thereby inhibiting G17-dependent tumor cell growth.

Furthermore, the use of anti-G17 immunization in combination with standard chemotherapy increases the efficacy of colorectal cancer treatment, since in the combination, lower amounts of chemotherapeutic agents may be required to treat a patient, thereby lowering their toxic effects on normal tissues. In addition, the patient's quality of life may be improved and his survival time prolonged.

In a preferred embodiment, the method comprises the active immunization of a mammal possessing a gastrin-dependent tumor with an anti-G17 immunogen, in combination with the administration of one or more chemotherapeutic agents, such as 5-fluorouraciL leucovorin, levamisole. cisplatin, tumor necrosis factor and proglumide. The anti-G17 immunogen may be administered to a patient at the onset of therapy and at subsequent intervals as required by the patient. The anti-G 17 antibodies produced by the patient following immunization bind and neutralize G17 in its mature, amidated-G17 as well as its precursor forms, e.g., G17-Gly, *in vivo* and prevent the binding of G17 to its receptors, thereby preventing gastrin-dependent tumor cell growth. The anti-G17 antibody titers produced by an immunized patient may be monitored at predetermined intervals using standard techniques. In addition, the chemotherapeutic agents may be administered as directed by standard regimes or lower doses may be administered as required by the patient.

In another embodiment, the invention further provides a method of treating a gastrin-dependent tumor comprising the passive immunization of a patient possessing a gastrin-dependent tumor with anti-G17 antibodies in combination with one or more chemotherapeutic agents, such as 5-fluorouracil leucovorin, levamisole, cisplatin, tumor necrosis factor and proglumide. In a preferred embodiment of this aspect of the invention, the antibodies may be chimeric. humanized, or human monoclonal antibodies which may be produced by methods well known in the art. The antibodies may be administered together with the chemotherapeutic agents at the onset of therapy and at subsequent intervals after the initial therapy, as required by the patient.

### BRIEF DESCRIPTION OF THE DRAWINGS

FIG. 1 depicts a graph showing a time scale of serum antibody titers after immunization of rats immunized with 500 µg/ml of rat anti-G17 (1-9)-DT immunogen.
FIG. 2 depicts a graph showing the effects of 30 mg/kg dose of 5-FU/leucovorin treatment on the anti-G 17( 1-9) antibody titers obtained in rats immunized with the immunogen of the invention.
**FIG.** 3 depicts a Scatchard plot showing the effects of treatment cycles of 30 mg/kg of 5-FU/leucovorin on the mean white blood cell counts in BDIX rats.
FIG. 4 depicts a bar graph showing the median tumor weight of untreated, anti-G17(1-9) DT-treated and DT-treated rats.
FIG. 5 depicts a bar graph showing the median tumor weights of rats treated with 30mg/kg of 5-FU/leucovorin;30 mg/kg of 5-FU/leucovorin and DT immunogen;30 mg/kg of 5-FU/leucovorin and anti-G17(1-9)-DT;25 mg/kg of 5-FU/leucovorin and DT immunogen;25 mg/kg of 5-FU/leucovorin and anti-G17(1-9)DT: 20 mg/kg of 5-FU/leucovorin and DT immunogen; 20 mg/kg of 5-FU/leucovorin and anti-G17(1-9)DT; 12.5 mg/kg of 5-FU/leucovorin and DT immunogen; and 12.5 mg/kg of 5-FU/leucovorin and anti-G 17(1-9)DT.

### DETAILED DESCRIPTION OF THE INVENTION

The present invention provides methods of treating tumors, in particular those associated with gastrin-dependent colorectal cancer, with a combination therapy comprising immunizing a patient with an anti-G17 immunogen and treating the patient with chemotherapeutic agents,such as 5-FU and leucovorin.The anti-G17 immunization/5-FU-leucovorin combination therapy, surprisingly,has been found to be more effective than previous therapies in treating colorectal cancer. The chemotherapeutic agents useful in the combination therapy do not significantly inhibit anti-G17 antibody production in an immunized patient and lower doses of chemotherapeutic agents can be used for treating the tumor growth. In addition, the anti-G17 antibody titers produced by immunization are effective to neutralize all forms of G 17 hormone.

In a preferred embodiment, the method comprises actively immunizing a patient afflicted with a gastrin-dependent colorectal cancer applying an anti-G17 immunogenic composition in conjunction with administering to the patient chemotherapeutic agents. Subsequent booster anti-G17 immunizations may be administered as required by the patient,as determined by analysis of the patient's serum anti-G17 antibody titers post-immunization using standard techniques and standard radiological assessments of the tumors. Anti-G17 immunization may also be provided to a patient prior to tumor surgery

The anti-G17 immunogens comprise a natural or synthetic peptide fragment of the N-terminal amino acids of G17 as the immunomimic portion of the immunogen. This peptide fragment is conjugated to an immunogenic carrier such as Diphtheria toxoid (DT).In a preferred embodiment of this aspect of the invention, the anti-G17 immunogen comprises the aminoterminal amino acids of G17 from positions 1 through 9, having the amino acid sequence pyroGlu-Gly-Pro-Trp-Leu-Glu-Glu-Glu-Glu, conjugated to Diphtheria toxoid. Other suitable immunogenic protein carriers, include bovine serum albumin, keylimpet hemocyain, hemocyanin and tetanus toxoid.

The immunogens of the invention may also comprise an extension or a spacer peptide sequence suitable for projecting the immunomimic peptide away from the protein carrier and for enhancing its capacity to bind the lymphocyte receptors. A suitable spacer peptide sequence is the amino acid sequence SSPPPPC (SEQ ID NO.: 2 in the Sequence Listing). However, other spacer peptides would be suitable as well. In a preferred embodiment of this aspect of the invention, the preferred spacer sequence is attached to the carboxy-terminal end of the immunomimic peptide. The immunogens of the invention are produced by standard techniques and are disclosed in U.S. Pat. Nos. 5,023,077; 5,468,494; 5,607,676; 5,609,870; 5,688,506 and 5,662,702. Following immunization, the immunogens of the invention produce high affinity, neutralizing antibodies for inhibiting the effects of G 17 in its mature and precursor forms on tumor growth in immunized animals.The anti-G17 antibodies produced bind and neutralize mature and precursor G17, thereby preventing the binding of G 17 to the receptors on tumor cells and ultimately inhibiting tumor cell growth. The immunogens raise antibodies which neutralize both the carboxy-amidated and glycine-extended G17, and show no cross-reactivity with G34 or CCK.

The compositions in which the immunogens for active immunization are administered for the treatment of gastrin-dependent tumors in patients may be in a variety of forms. These include, for example, solid, semi-solid and liquid dosage forms, such as powders, liquid solutions, suspensions, suppositories, and injectable and infusible solutions.The preferred form depends on the intended mode of administration and therapeutic applications. The compositions comprise the present immunogens and suitable pharmaceutically acceptable components, and may include other medicinal agents, carriers, adjuvants excipients, etc., which can be mixed using standard procedures. Preferably, the compositions are in the form of unit doses. The amount of active compound administered for immunization or as a medicament at one time, or over a period of time, will depend on the subject being treated, the manner and form of administration, and the judgment of the treating physician.

An effective dosage ranging from 0.001 to 2 mg of the immunogenic composition is administered to the patient for the treatment of the gastrointestinal cancer. The effective dosage of the immunogenic composition is capable of eliciting an immune response in a patient of effective levels of antibody titer to bind and neutralize mature and precursor G17 for 1-3 months after immunization. Following the immunization and the chemotherapeutic agent treatment, with, for example, 5-FU/Leucovorin, of a patient with colorectal cancer, the effectiveness of the therapy on tumor growth is assayed by standard clinical procedures, such as ultrasound and magnetic resonance imaging (MRI) to detect the presence and size of tumors, if any. The anti-G 17 antibody titers may also be monitored from a sample of blood taken from the patient.

Booster immunizations should be given as required to maintain an effective antibody titer Effective treatment of gastrin-dependent colorectal adenocarcinoma and other gastrin-dependent cancers such as stomach, liver, pancreatic and small cell carcinoma of the lungs according to this method should result in inhibition of tumor growth and a decrease in size of the tumor.

For passive immunization, the anti-G17 antibodies are administered to a patient intravenously using a pharmaceutically acceptable carrier, such as saline solution, for example, phosphate-buffered saline.

The chemotherapeutic agents are administered at doses recommended in standard regimes and may be administered at the onset of therapy simultaneously with anti-G17 immunogen, prior to immunization or after immunization. In some cases, it may be beneficial to administer the chemotherapeutic agent both before and after immunization. Subsequent chemotherapeutic treatments may also be administered as required by the patient following evaluation by MRI and ultrasound imaging.

The following experiments were conducted to demonstrate the effects of the present combination therapy on colorectal cancers.

### EXAMPLE 1

The following experiments were conducted to determine the potential clinical benefit offered by anti-G 17(1-9)-DT. The aims of this study were as follows:
(a) to determine the long term effect of specific rat anti-G 17(1-9)-DT immunization on the histological appearance of the rat GI tract.
(b) to evaluate the effect of 5-FU/Leucovorin combinations on antibody titers raised by anti-G 17(1-9)-DT; and
(c) to determine the therapeutic effect of anti-G17(1-9)-DT and 5-FU/leucovorin combinations on a rat colon model.

### Cell line

DHDK12 is a rat colonic epithelial tumor cell line (Martin, 1983). The cell line was maintained in RPMI 1640 growth medium (Gibco, Paisley, Scotland) containing 10% fetal calf serum (FCS, Sigma, Poole, UK) in humidified conditions at 37°C and 5% CO₂.

### Immunogen

The anti-G17(1-9)-DT immunogen consists of amino acid residues 1-9 of G 17 linked via the carboxy-terminus to the peptide spacer SSPPPPC (SEQ ID NO.: 1 in the Sequence Listing). which in turn is conjugated to DT. The immunogen used in these studies was made specific for rat G17 by replacing the human G17 epitope with the amino terminal 9 amino acids of rat G17. linked through a peptide spacer to diphtheria toxoid (DT). Antiserum raised by rat anti-G17(1-9)-DT was denoted as anti-rat G17 (1-9):DT.

### Experimental animals

Male and female BDIX rats were provided by the Cancer Studies Unit, University of Nottingham. UK and were 6-10 weeks old, weighing 340-420 g. The rats were housed in pairs and maintained in a cycle of 12-hour light and 12-hour dark at 25°C with 50% humidity.

Prior to each experiment, the animals were grouped to equalize weight distribution. Group sizes ranged from 6-13 animals. The UK Coordinating Committee for Cancer Research (UKCCCR) guidelines were adhered to throughout all animal experimentation.

### Immunization procedure

Rat anti-G17(1-9)-DT was dissolved in sterile saline (0.9%), pH 7.3 to 1mg/ml. The adjuvant, nor-muramyl dipeptide (Peninsula Labs., Belmont, CA, USA), was added to the conjugate solution to give a final conjugate concentration of between 200 and 500 µg/ml. The aqueous solution was formulated with an oily vehicle (montanide ISA 703: AMS Seppic Inc., Paris, France) in a 1:2 ratio (v/v) by emulsification. After placing in a glass syringe which was attached to a second syringe through a three-way stopcock, the mixture was forced back and forth through the syringes 40 times to form an emulsion. An emulsion containing DT peptide and muramyl dipeptide was similarly formulated for control rats. A 200µl volume of emulsion (50 µg/rat) was injected s.c. (right hand flank of the experimental animals). The animals were immunized with either a single injection or repeatedly at 21 day intervals as detailed below

### Cytotoxic treatment regime

Rats received 12.5 and 25 mg/kg of 5-Fluorouracil (5-FU, David Bull Labs., Warwick. UK) and 12.5-25 mg/kg Leucovorin (Lederle Labs.. Gosport. Hants, UK) administered intravenously (iv) on days 1, 3 and 5 with the cycle being repeated every 4 weeks over the duration of the study period (Asao, 1992). The cytotoxic combination was administered to the rats either prior to or after anti-G17(1-9)-DT immunization (200 µg/ml).

### Initiation of tumor growth

DHDK12 cells were suspended in sterile phosphate buffered line (PBS, Oxoid, Hants., UK) at a cell concentration of 2.5x10⁷/ml. Rats were anesthetized by a 1 ml intraperitoneal injection of Hypnorm (0.315 ng/ml fenatanyl citrate and 10 mg/ml fluanisone; Jannsen, Berrse, Belgium), Hypnovel (5 ng/ml midazolam; Roche, Basel, Switzerland), and sterile distilled water in a 1: 1:5 ratio. Following a subcutaneous (s.c.) incision on the right flank, a 200 µl volume of cell suspension was injected into the muscle layer of the abdominal wall and the surgical incision closed by wound clips. Each experimental group was composed of between 6 and 13 animals.

### Determination of specific antibody levels of rat anti-G17(1-9)-DT-immunized rats

To obtain blood samples for analysis, rats were tail-bled at various time points throughout the experiment and at termination by cardiac puncture under terminal anesthesia. Serum anti-rat-G17 antibody levels were determined by enzyme-linked immunosorbent assay (ELISA). A rat G17-bovine serum albumin (BSA) conjugate was dissolved to 2 µg/ml in 0.1M glycine buffer (pH 9.5) and 25µl per well was plated into 96-well Immunulon U plates (Dynatech Labs., Sussex, UK). The wells were incubated overnight at 4°C after which the unadsorbed conjugate was flicked out and the wells were washed with buffer (0.9% saline, 0.5% Tween-20 [Sigma], 0.02% NaN₃ [Sigma], pH 7.3). This buffer was used for all washing steps and reagent dilutions. Sera were treated at 10-fold serial dilutions, starting at a dilution of 1: 100. The positive control was rat anti-rat G 17(1-9)-DT antiserum from previously immunized animals and the negative controls were normal rat serum, and serum from rats immunized with DT only. All control sera were used at the same dilutions as the test sera. The diluted sera were added to the wells in 25 µl aliquots in the presence or absence of 25 µl/well rat G17-BSA at 100 µg/ml (as a soluble inhibitor). Baseline control wells received 25 µl assay buffer only. The plates were incubated for 60 minutes. at room temperature before washing with the assay buffer. Goat anti-rat immunoglobulin (H+L)-biotin (Zymed, San Francisco, CA, USA) was added to the wells at a 1:500 dilution, 50 µl/well and incubated for 60 minutes, in the dark at room temperature After washing, avidin-alkaline phosphatase (Zymed), 1: 100 dilution was added (50 µl/well ) and the plates were incubated for 60 mins. at room temperature. After further washing, p-nitrophenylphosphate (pNPP) substrate (Sigma) was added to the wells at 50 µl/well and after a 5-minute developing time, the absorbance was read at 405 nm. The difference in absorbance between untreated sera and sera co-incubated with rat G17-BSA was calculated as the specific absorbance.

### Determination of white blood cell counts

Heparinized blood from the rats was collected by tail bleeds during the experiment and by cardiac puncture at the termination of the experiment. The numbers of white blood cells were analyzed by the Hematology Department at the University Hospital, Nottingham with the use of a FaCScan.

### Histology

At termination of the long-term anti-G17(1-9)-DT-immunized rats, representative areas of the stomach, colon and rectum from the immunized rats and age-matched controls were dissected and formalin-fixed. The sections were then embedded in paraffin and 4 µm sections were cut by use of a microtome. These were stained by hematoxylin and eosin and evaluated by a histopathologist who had no knowledge of the treatment groups.

### Crypt cell proliferation rate

One hour prior to animal termination. vincristine (2 mg/kg, Sigma) was injected intraperitoneally to induce metaphase arrest in the colonic epithelium prior to the assessment of colonic crypt cell proliferation (CCPR). The number of cells in metaphase per crypt were counted. The colon and rectum were removed from each rat, opened longitudinally and mucosa from each fixed in Carnoys' solution. Crypts were gently squashed, longitudinally, under a dissecting microscope and the number of cells in metaphase enumerated (magnification x 25).

### Statistical analysis

*In vivo* results were analyzed by a Mann Whitney non-parametric test by use of the SPSS statistical package for the IBM PC.

### Long term anti-G17(1-9)-DT studies

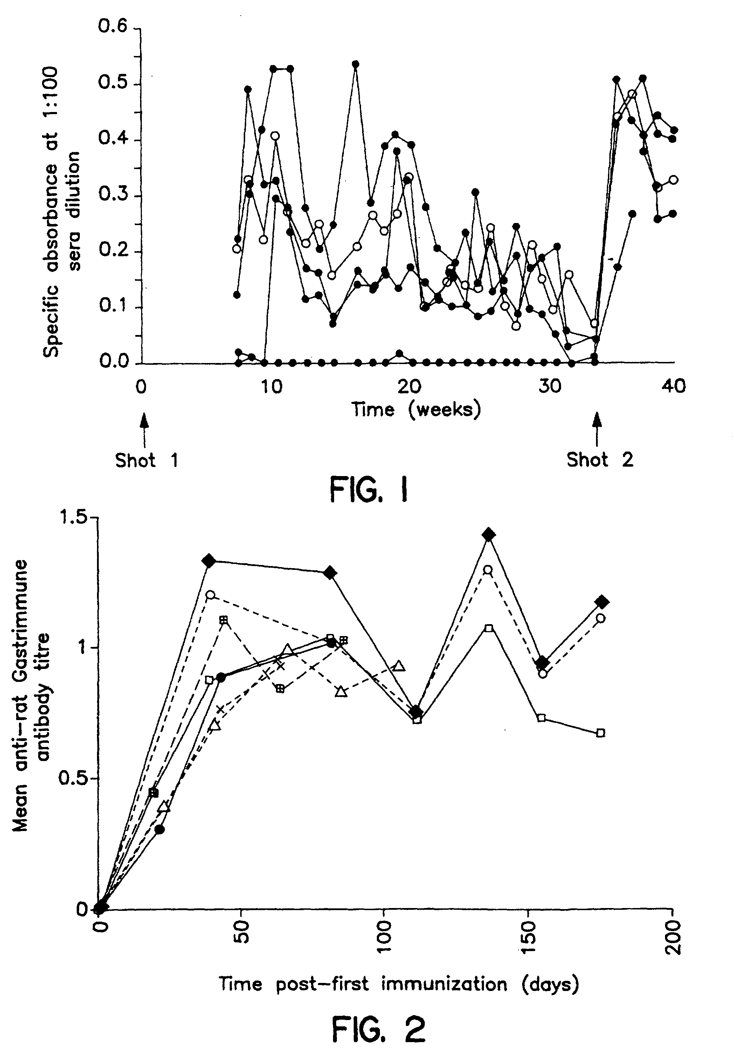

Five male rats were immunized with rat anti-G17( 1-9)-DT immunogen as described above, and their antibody titers were measured for a period of 34 weeks following a single immunization. At this point, the rats were boosted with a second injection of rat anti-G 17( 1-9)-DT. The results are shown in FIG. 1. FIG. 1 shows the time-scale up to 40 weeks after immunization of antibody titers from rats immunized with 500 µg/ml of rat anti-G 17(1-9)-DT. Each point represents an individual animal. Antibody titers were measured by an ELISA assay as described above using a 1:100 dilution of sera. Immunizations are indicated by the arrow. Following the primary immunization, 4 of the 5 rats responded to the rat anti-G 17( 1-9)-DT immunogen. Antibodies, following this single injection, were detectable by week 7 in 3 of 5 rats and in 4 of 5 rats by week 9. This initial surge of antibodies was followed by a second surge between 15-20 weeks, after which the antibody titers steadily declined and were approaching zero by week 34. At this point, FIG I also shows that after a second immunization with rat anti-G17(1-9)-DT, all rats had detectable anti-rat-G-17 antibody titers within 1-2 weeks post-immunization.

### EXAMPLE 2

### Histological analysis of the long term anti-G17(1-9)-DT-immunized rats

Specimens from the stomach, colon and rectum were evaluated histologically following hematoxylin and eosin staining as described in EXAMPLE 1. These were compared to specimens from age and sex-matched control rats. All areas of the GI tract evaluated were identical in both anti-G17(1-9)-DT-treated and age-matched control rats with respect to length of villae/crypts/mucosal height. In the stomach, enterochromaffin-like (ECL) cells were similar in number and appearance in the two subject animal groups. However, there was some evidence of granulation of the G cells in the anti-G 17( 1-9)-DT-treated rat stomach mucosa.

### EXAMPLE 3

### Crypt cell proliferation rate (CCPR) of colonic epithelium from long term anti-G17(1-9)-DT-immunized rats

The CCPR of colonic epithelium and anti-rat G17-antibody titers were analyzed as described above. Table I shows the results obtained from 4 of 5 rats evaluated comparing CCPR to anti-rat G17 antibody titers. The mean CCPR for control rats was 18.93 (standard deviation 3.2) and for the anti-G17( 1-9)-DT-immunized rats 23.7 (standard deviation 7.9). There was no statistical difference in CCPR between the anti-G17(1-9)-DT-immnnized and age-matched control rats. These results indicate that the rate of crypt cell division in a colonic epithelium is the same for control and anti-G17(1-9) DT-immunized rats.

**Table I**

| **A comparison of anti-rat G17: DT antibody titers with the crypt cell proliferation of the colon** | | |
|---|---|---|
| **Rat** | **Specific absorbance relating** **to anti-rat G17:DT** **antibodies, (1:1000 dilution)** | **Crypt cell proliferation rate** **(mean metaphases/crypt after 2 hours vincristine treatment)** |
| Control 1 | 0 | 21.9 |
| Control 2 | 0 | 19.4 |
| Control 3 | 0 | 14.4 |
| Control 4 | 0 | 20.0 |
| Immunized rat 1 | 0.280 | 29.7 |
| Immunized rat 2 | 0.340 | 30.3 |
| Immunized rat 3 | 0.415 | 13.6 |
| Immunized rat 4 | 0.420 | 21.1 |

### EXAMPLE 4

### Effect of pre- and post-cytotoxic treatment on antibody levels raised by rat anti-G17(1-9)-DT

Rats were injected intravenously with a 1:1 ratio of 5-FU/Leucovorin at 30 mg/kg as described in Example 1 prior to or after anti-G17 (1-9)-DT immunization. Each group consisted of 6 male and 6 female rats per group and the mean antibody titers were measured by an ELISA technique using a 1: 100 dilution of sera. Antibody levels in each rat were measured from blood samples as described in Example 1.

FIG. 2 shows the effect of pre- and post-cytotoxic treatment with 30 mg/kg of 5-FU/Leucovorin cycles on antibody titers raised by anti-G17(1-9)-DT immunization(500 µg/ml). In the Figure, the data is represented as follows: -□- no cytotoxics, 7 immunizations; -◆- 2 immunizations prior to 4 cytotoxic treatments; -○- 1 immunization prior to 4 cytotoxic treatments; -Δ- 1 cytotoxic prior to 4 immunizations (2 cytotoxic treatments during immunizations); - 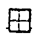 - 2 cytotoxic treatments prior to 4 immunizations; -∗- 3 cytotoxic treatments prior to 3 immunizations; and -•- 4 cytotoxic treatments prior to 2 immunizations.

FIG. 2 shows the mean of 6 female and 6 male rats per group. The standard deviations were around 10% of the mean. There was no significant effect on antibody titers by pre-treatment with the cytotoxic 5-FU/Leucovorin combination on either the antibody levels achieved or the time taken to achieve those levels when compared to untreated anti-G 17(1-9)-DT-immunized rats. The maximum number of treatment cycles evaluated was 4 cytotoxic treatment cycles followed by 2 immunizations.

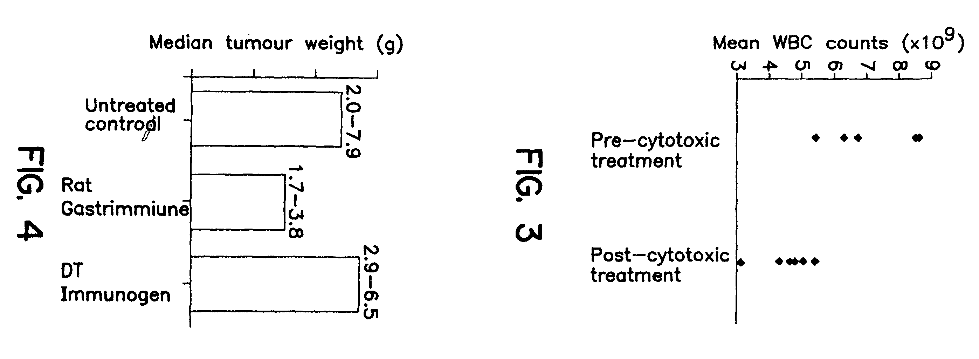

Figure 3 shows the effects of treatment on mean white blood cells (WBC) counts, in BDIX rats.

The effect of cytotoxic treatment with 30 mg/kg 5-FU/Leucovorin in BDIX rats receiving 4 cytotoxic treatments prior to 2 immunizations on the mean white blood cell (WBC) counts is shown in FIG. 3. As shown in the Figure, there was a significant reduction in WBC counts in the representative rats evaluated, post cytotoxic treatment (p<0.005, Students' t-test). The counts were reduced by the number of cytotoxic treatment cycles, indicative of some myelosuppression. However, there was no effect on the antibody response to anti-G17(1-9)-DT produced by the rats, as shown in FIG. 2.

### EXAMPLE 5

### Effect of combination therapy of 5-FU Leucovorin and anti-G17(1-9)-DT on the in vivo growth of DHDK12 tumors

The effects of combined therapies with 5-FU/Leucovorin (12.5-30 mg/kg) and rat anti-G37(1-9)-DT (200µg/ml) on the growth of the rat colon tumor DHDK12 cell line in the muscle layer of the abdominal wall of BDIX rats were tested by comparison to tumors in control animals as described in the previous Examples. At the end of the therapies the rats were killed, their tumors excised and weighed using standard procedures. Each group consisted of 10-12 rats/group of mixed sex. The median tumor weights are shown with the inter-quartile ranges above the columns. Statistical assessment was done by a Mann Whitley U non-parametric test as described in Example 1.

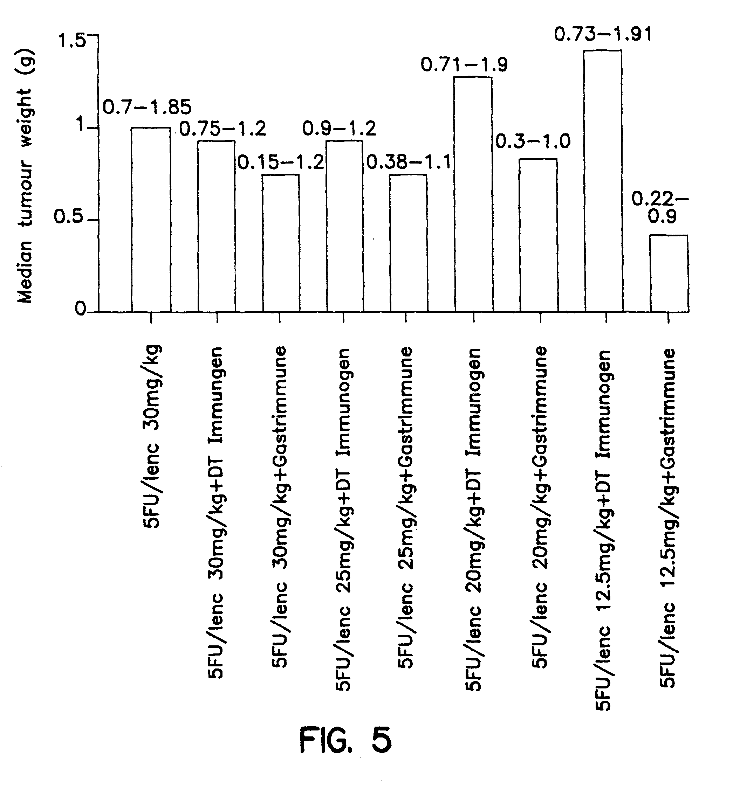

FIGs. 4 and 5 show the effect of anti-G 17(1-9)-DT immunization on the median final tumor weights from BDIX rats implanted with DHDK12 tumor cells in the muscle layer of the abdominal wall. This route of implantation results in a well-vascularized tumor amenable to therapies administered into the circulation (Watson, 1996). Rat anti-G17(1-9)-DT had previously been shown to inhibit final DHDK12 tumor weight by 56.5% when administered at a dose of 500 µg/ml (Watson, 1996). In the present experiments, to detect any benefits of combination therapy with 5-FU/Leucovorin, the anti-G17(1-9)-DT dose was dropped to 200 µg/ml, which resulted in a significant inhibition of tumor growth of 25.7% as shown in FIG. 4. FIG. 4 shows data from tumors excised from untreated control rats, anti-G17(1-9)-DT immunized rats and DT-immunized rats. After a 50 day time period untreated rats had a median tumor weight of 4.43 g. DT immunization resulted in a median tumor weight of 4.7 g. which was not significantly different from the tumor weights of untreated rats. but which was significantly greater than the median tumor weight of anti-G 17(1-9)-DT-immunized rats (3.49 g, p=0.034, Mann Whitney).

FIG. 5 shows that 5-FU/Leucovorin alone, given at 30 mg/kg, significantly reduced tumor weight to a median of 1.01 g (p=0.0106 when compared to untreated control rats). When rats were treated with the same cytotoxic dose of 5-FU/Leucovorin together with DT immunization, the median tumor weight was not significantly different (0.945 g).

A combination of 5-FU/Leucovorin at 30 mg/kg and rat anti-G17(1-9)-DT immunization resulted in a median tumor weight of 0.68 g which was not significantly different from the 5-FU/Leucovorin/DT-treated group (p=0.27). The combination of 25 mg/kg 5-FU/Leucovorin and DT immunization resulted in a median tumor weight of 0.96 g compared to a mean tumor weight of 0.68g in the anti-G 17( 1-9)-DT-immunized in conjunction with 5-FU/Leucovorin combination therapy group which was not significant (p=0.409). When the 5-FU/Leucovorin dose was reduced to 20 mg/kg, the 5-FU/Leucovorin/DT immunogen combination resulted in a median tumor weight of 1.23 g. The median tumor weight was significantly reduced to 0.71 g when 20 mg/kg of 5FU/leucovorin was combined with anti-G 17( 1-9)-DT immunization (p=0.027, Mann Whitney).

Finally, FIG 5 also shows that a 5-FU/Leucovorin dose of 12.5 mg/kg combined with anti-G17(1-9)-DT immunization (p=0.015, Mann Whitney) reduces the median tumor weight from 1.34 g to 0.41 g. 5-FU/Leucovorin-anti-G17(1-9)-DT combinations were compared and no statistically significant difference existed between anti-G17(1-9)-DT given in combination with either 12.5. 20 or 30 mg/kg of 5-FU/Leucovorin.

Due to the limited benefit shown for combination chemotherapy with 5-FU/Leucovorin in both an advanced cancer state and, in particular, with an adjuvant therapy treatment setting (Moertel, 1994; Scheithauer, 1995; Taylor, 1993; Petrioli. 1995). new therapeutic modalities may need to be given either in conjunction with 5-FU/Leucovorin to enhance the therapeutic index (and possibly reduce the chemotherapeutic dose to limit toxicity) or as a second line treatment if chemotherapy fails to be effective. Thus new treatments must be amenable for such use. immunotherapeutic approaches in conjunction with chemotherapy were previously thought to be problematic due to the myelosuppression associated with chemotherapeutic agents, such as that seen with 5-FU/Leucovorin (Mahood. 1991). In the present study, however, myelosuppression of rats induced with 5-FU/Leucovorin combinations of 30 mg/kg. administered according to Asao *et al.* at the maximum tolerated dose. did not affect the level of and time to achieve anti-rat G17:DT antibody titers following immunization with the anti-G17(1-9)-DT immunogen.

In therapy studies using 5-FU/Leucovorin in combination with anti-G17(1-9)-DT, a potentiation of the 20 mg/kg and 12.5 mg/kg dosages was achieved. The 20 mg/kg dose was as effective as the maximum tolerated dose when combined with anti-G17(1-9)-DT, and the 12.5 mg/kg dose showed a trend to a greater therapeutic effect. The reason for the latter trend is not known but it may be due to the cytotoxic dose affecting the immune system to a lesser degree than higher dose levels, which may aid in the general inflammatory response against the tumor. 5-FU/Leucovorin given in continuous cycles would appear to exert an 'all or nothing' effect on tumor growth as lowering the dose to 1 mg/kg was found to exert no inhibition of tumor growth. The therapeutic effect may be titrated out more gradually by reducing the number of toxic cycles (Watson. personal communication). Therefore, in the combinations according to the present invention, lower than usual doses of 5-FU/Leucovorin can be administered, thus reducing the side effects of the drugs, while, at the same time, effective killing of tumor cells can be achieved using the present combination, since the immune system is only minimally affected. Thus, the growth inhibitory effect of anti-G17(1-9)-DT immunization is enhanced. These characteristics of the combination therapy are unexpected and surprising in view of the myelosuppressive effects of the chemotherapeutic agents by themselves.

Furthermore, by the absence of deleterious effects on the host, anti-G17( 1-9)-DT immunization is likely to be a long-term treatment as shown by the length of time that measurable antibody levels were present in rats receiving a single immunization. The first immunization was shown to be 80% effective, in terms of anti-gastrin antibody induction, and 100% effective after the second immunization with an immediate rise in antibody levels. Although potentiation of chemotherapy may be achieved by a single anti-G17(1-9)-DT injection. in most hosts the absence of side effects, characteristic of anti-G 17(1-9)-DT immunization, and the host response rate following boosts, indicate a multi-injection regime may be desirable. Despite the length of time that anti-rat-G17 antibodies remained in the circulation there appeared to be no long term deleterious effects on the GI tract, as determined by a simple histological assessment. Additionally, the crypt cell proliferation index of mucosal cells in the colon revealed no significant effect on their growth.

### Example 6

### Treatment of human colon cancer patients with a combination therapy of 5-FU/Leucovorin and anti-G17 (1-9)-DT.

Anti-G17(1-9)-DT immunization alone has previously been shown to be a valuable and safe therapeutic option in the treatment of gastrin-dependent cancer. The present combinations of anti-G17 immunogens with 5-FU/Leucovorin enhance the effectiveness of cancer treatment,in particular colon cancer treatment,and the possible reduction in the dosage of the chemotherapeutic agent required in the combination should reduce the deleterious cytotoxic side effects of any of the chemotherapeutic agents now in use. The present combinations of an immunogen with chemotherapeutic agents may also be useful as a second-line therapy in patients who do not respond to chemotherapy alone.

Human colorectal tumor or colon cancer patients are treated with a combination of chemotherapy and immunotherapy.

Specifically,for patients with gastrin responsive colorectal tumors or colon cancer can be treated with concomitant administration of 5-FU/Leucovorin and an anti-G17 immunogen composition or anti-G17 antibodies.

In particular, the preferred immunotherapy provides an immunogenic composition comprising an aminoterminal G17 (1-9) peptide: DT conjugate in a pharmaceutically acceptable carrier which may include an adjuvant to further stimulate the immune response.

The preferred immunotherapeutic regimen can start before, during or after the chemotherapy course depending on clinical considerations. For example, in a patient with a large tumor burden it may be advantageous to start with several cycles of chemotherapy to reduce the tumor bulk and then start with immunotherapy.

Alternatively, in a patient with a small tumor burden or after curative surgery, immunotherapy can be started before or during chemotherapy.

The active immunization dose can range between 300 µg up to 1200 µg of the anti-G17 immunogen, depending on the immune status of the patient (or the capacity of an immune response). The injection intervals can be on days 1, 7 and 14, or days 1, 14 and 21, or days 1, 14, then 28 and 56. All the schedules can result in similar antibody titers. The accelerated schedules of immunization provide the possibility of earlier onset of immune response.

The preferred method of the anti-gastrin therapy provides that a booster is administered every 6 months after the initial immunization period, regardless of which protocol is used.

Yet another preferred method for the effective neutralization of G17, Gly G17 and G17 NH₂ provides passive immunization with anti-G 17 antibodies, preferably in purified form.More specifically, the inoculation of 10-1000 µg anti-G17(1-9) antibodies is administered before, during and/or after the chemotherapy cycles for the control of gastrin activities. The passive immunization can be administered daily, weekly or biweekly. Other protocols can be followed depending on the effectiveness of the treatment.

A further combination of treatment provides for an initial passive immunization before and/or during the first cycle of chemotherapy followed by active immunization as described above.

Many chemotherapy regimens are in use. These art recognized regimens, although not described herein, are not excluded from the combination treatment according to this invention.One preferred chemotherapy regimen provides for 5-FU i.v. bolus of 425 mg/m² with i.v. infusion of Leucovorin (folic acid, FA, 20 mg/m²) for 1-5 days per period up to 4 weeks.

Another preferred regimen provides for 200 mg/m² FA over a period of 2h, followed by 5-FU i.v. boles of 400 mg/m² + 5-FU of 600 mg/m² over 22 hours 1 or 2 days in a 2-week period.

Yet another preferred regimen provides for continuous infusion of 5-FU at 250-300 mg/m² day continuous i.v. for 4-6 weeks, followed by 2 weeks rest.

### REFERENCES

1. Dickinson, C.J. And Yamada, T. Gastrin-amidating enzymes in the porcine pituitary and antrum. Characterization of molecular forms and substrate specificity. *J. Biol Chem.* **266**(1): 334-338, 1991.
2. Dickinson, C.J. Relationship of gastrin processing to colon cancer. *Gastroenterology,* **109**: 1384-1388, 1995.
3. Ciccotosto, G.D., McLeish, A., Hardy. K.J., and Shulkes, A. Expression, processing, and secretion of gastrin in patients with colorectal carcinoma. *Gastroenterology,* **109**: 1142-1153, 1995.
4. Kopin, A.S.; Lee, Y.M.; McBride, E.W.; Miller, L.J.; Lu, M.; Lin, H.Y.; Kolakowski, L.F., Jr., and Beinborn, M. Expression cloning and characterization of the canine parietal cell gastrin receptor. *Proc. Natl. Acad. Sci. USA* **89(8)**: 3605-3609, 1992.
5. Edkins, J.S. On the chemical mechanism of gastrin secretion. Proc. R. Soc. London, 76:376,1905.
6. Johnson, L.R., Wang, P. And Haddox, K. Omithine decarboxylase in large bowel mucosa: regulation by gastrin, secretin and EGF. *J. Physiol. Pharmacol.* **43(1)**: 33-41, 1992.
7. Watson, S.; Durrant, L., and Morris, D. Gastrin: growth enhancing effects on human gastric and colonic tumour cells. *Br. J. Cancer,* **59(4)**: 554-558, 1989.
8. Rehfeld, J.F.; Bardram, L. And Hilsted, L. Gastrin in human bronchogenic carcinomas: constant expression but variable processing of progastrin. *Cancer Res.* **49(11)**: 2840-2843, 1989.
9. Rehfeld, J.F. Three components of gastrin in human serum. *Biochim. Biophys. Acta.,* **285**: 364-372, 1972.
10. Upp, J.R., Singh, P., Townsend, C.M., Jr. and Thompson, J.C. Clinical - significance of gastrin receptors in human colon cancers. *Cancer Res.* **49(2)**: 488-492, 1989.
11. Watson, S.A., and Steele, R.J.C. *Gastrin receptors in GI tumors.* R.G. Landes, Austin, USA 1993.
12. Van-Solinge, W.W., Nielsen, F.C., Friis-Hansen, L., Falkmer, U.G., and Rehfeld, J.F. Expression but incomplete maturation of progastrin in colorectal carcinomas. *Gastroenterology,* **104**: 1099-1107,1993.
13. Nemeth, J., Taylor, B., Pauwels, S., Varro A, and Dockray, G.J. Identification of progastrin derived peptides in colorectal carcinoma extracts. *Gut,* 34: 90-95, 1993.
14. Seva, C., Dickinson, C.J., and Yamada, T. Growth-promoting effects of glycine-extended progastrin. *Science,* **265**: 410-412, 1994.
15. Finley, G.G., Koski, R.A., Melham, M.F., Pipas, J.M., and Meisler, A.I. Expression of the gastrin gene in the normal human colon and colorectal adenocarcinoma. *Cancer Res.,* **53**: 2919-2926, 1993.
16. Kochman, M.L., Del Valle, J., Dickinson, C.J. and Boland, C.R Post-translational processing of gastrin in neoplastic human colonic tissues. *Biochem. Biophys. Res. Commun.* **189(2)**: 1165-1169, 1992.
17. Kelly, A. Hollande, F., Shulkes, A. And Baldwin, G.S. Expression of progastrin-derived peptides and gastrin receptors in a panel of gastrointestinal carcinoma cell lines. *J. Gastroenterol. Hepatol.* **13(2)**: 208-214, 1998.
18. Hoosein, N.M., Kiener, P.A., Curry, R.C., and Brattain, M.G. Evidence for autocrine growth stimulation of cultured colon tumor cells by a gastrin/cholecystokinin-like peptide. *Exptl. Cell Res.,* **186**: 15-21, 1990.
19. Baldwin, G.S., and Zhang, Q-X. Measurement of gastrin and transforming growth factor a messenger RNA levels in colonic carcinoma cell lines by quantitative polymerase chain reaction. *Cancer Res.,* **52**: 2261-2267, 1992.
20. Hoosein, N.M., Kiener, P.A, and Curry, R.C. Anti-proliferative effects of gastrin receptor antagonists and antibodies to gastrin on human colon carcinoma cell lines. *Cancer Res.,* **48**: 7179-7183, 1988.
21. Watson, S.A., Durrant, L.G., Wencyk, P.M., Watson, A.L., and Morris, D.L. Intracellular gastrin in human gastrointestinal tumor cells. *J.N.C.I.,* **83**: 866-872, 1991 a.
22. Varro, A., Henry, J., Vaillant, C., and Dockray, G.J. Discrimination between temperature- and brefeldin A-sensitive steps in the sulfation, phosphorylation, and cleavage of progastrin and its derivatives. *J. Biol. Chem.* **269(32)**: 20764-20770, 1994.
23. Hoosein, N.M., Kiener, P.A., Curry, R.C., and Brattain, M.G. Evidence for autocrine growth stimulation of cultured colon tumor cells by a gastrin/cholecystokinin-like peptide. *Exp. Cell Res.* **186(1):** 15-21, 1990.
24. Watson, S.A., Durrant, L.G., Elston, P., and Morris, D.L. Inhibitory effects of the gastrin receptor antagonist (L-365,260) on gastrointestinal tumor cells. *Cancer* **68(6)**: 1255-1260, 1991(b),
25. Yamada, T., Chiba, T., Del Valle, J. and Campbell, V. Postreceptor signals that mediate gastrin action on gastric parietal cells. In: Gastrin, J. A Walsh ed., Raven Press, New York, pages 151-168, 1993.
26. Todisco, A., Takeuchi, Y., Seva, C., Dickinson, C.J., and Yamada, T. Gastrin and Glycine-extended progastrin processing intermediates induce different programs of early gene activation. *J. Biol. Chem.* **270(47)**: 28337-28341, 1995.
27. Taniguchi, T., Matsui, T., Ito, M., Murayama, T., Tsukamoto, T., Katakami, Y., Chiba, T., and Chihara, K. Cholecystokinin-B/gastrin receptor signaling pathway involves tyrosine phosphorylations of p125FAK and p42MAP. *Oncogene* **9(3)**: 861-867, 1994.
28. Moertel, G.G. Chemotherapy CRC. *NEJM* 1994; **330**: 1136-1142.
29. Petrioli, R., Lorenzi, M., Aquino, A., Marsili, S., Frediani, B., Palazzuoli, V., Marzocca, G. Treatment of advanced colorectal cancer with high-dose intensity folinic acid and 5-Fluorouracil plus supportive care. *Eur J Cancer* 1995; **31A**: 2105-2108.
30. Scheithauer, W., Kornek, G., Rosen, H., Sebesta, C., Marcell, A., Kwasny, W., Karall, M., Depisch, D. Combined intraperitoneal plus intravenous chemotherapy after curative resection for colonic adenocarcinoma. *Eur J Cancer* 1995; **31A**: 1981-1986.
31. Taylor, I. Chemotherapy, radiotherapy and immunology of colorectal neoplasia. *Current opinion in Gastroenterology* 1993; **9**: 28-33.
32. Mahood, D.J., Dose, A.M., Lopniz, C.C. Inhibition of fluorouracil stomatitis by oral cryotherapy. *J Clin Oncol* 1991; **9**: 449-452.
33. Erlichman, C., Fine, S., Wong, A., Elhakeim, T. A randomized trial of fluorouracil and folinic acid in patients with metastatic CRC. *J Clin Oncol* 1988; **6**: 496-475.
34. Pietnelli, N., Douglas HO., Harrava, L. The modulation of Fluorouracil with leucovorin in metastatic CRC: a prospective randomized phase III trial. *J Clin Oncol 1989;* **7**: 1419-1426.
35. Seva, C., Dickinson, C. J., Sawada, M., Yamada, T. Characterization of the glycine-extended gastrin (G-gly) receptor on AR4-2J cells. *Gastroenterol 1995;* **108**: A742.
36. Harrison, J.D., Jones, J.A., and Morris, D.L. The effect of the gastrin receptor antagonist proglumide on survival in gastric carcinoma. Cancer **66(7)**: 1449-1452, 1990.
37. Bock, M.G., DiPardio, R. M., Evans, B.E., Rittle, K.E., Whitter, A., Veber, D, Anderson, E., and Freidinger, A. Benzodiazepine, gastrin and brain cholecystokinin receptor ligands: L-365,260. *J. Med Chem.,* **32**: 13-17, 1989.
38. Hughes, J., Boden, P., Costall, B., Domeney, A, Kelly, E., Horwell, D.C., Hunter, J.C., Pinnock, R.D., and Woodruff, G.N. Development of a class of selective cholecystokinin type B receptor antagonists having potent anxiolytic activity. *Proc. Natl. Acad Sci.,* **87**: 6728-6732, 1990.
39. Romani, R., Howes, L.G:, and Morris, D.L. Potent new family of gastrin receptor antagonists (GRAs) produces *in vitro* and *in vivo* inhibition of human colorectal cancer cell lines. *Proc. AACR,* **35**: 397 (Abstract), 1994.
40. Yuki, H., Nishida, A., Miyake, A., Ito H., Akuzawa, S., Takinami, Y., Takemoto, Y., and Miyata, K. YM022, a potent and selective gastrin/CCK-B receptor antagonist, inhibits peptone meal-induced gastric acid secretion in Heidenhain pouch dogs. *Dig. Dis. Sci.* **42(4)**: 707-714, 1997.
41. Takinami, Y., Yuki, H., Nishida, A., Akuzawa, S., Uchida, A., Takemoto, Y., Ohta, M., Satoh, M., Semple, G., Miyata, K. YF476 is a new potent and selective gastrin/cholecystokinin-B receptor antagonist *in vitro* and *in vivo. Aliment. Pharmacol. Ther.* **11(1):** 113-120, 1997.
42. Watson, S.A., Morris D.L., Durrant, L.G., Robertson, J.F., and Hardcastle, J.D. Inhibition of gastrin-stimulated growth of gastrointestinal tumour cells by octreotide and the gastrin/cholecystokinin receptor antagonists, proglumide and lorglumide. *Eur. J Cancer.* **28A(8-9)**: 1462-1467, 1992a.
43. Watson, S.A., Crosbee, D.M., Morris, D.L., Robertson, J.F., Makovec, F., Rovati, L.C., and Hardcastle, J.D. Therapeutic effect of the gastrin receptor antagonist, CR2093 on gastrointestinal tumour cell growth. *Br. J. Cancer* **65(6):** 879-883, 1992b.
44. Seva, C., Scemama, J.L., Bastie, M.J., Pradayrol, L., Vaysse, N. Lorglumide and loxiglumide inhibit gastrin-stimulated DNA synthesis in a rat tumoral acinar pancreatic cell line (AR42J). *Cancer Res.* **50(18)**: 5829-5833, 1990.
45. Asao, T., Takayuki, A., Shibata, H.R., Batist, G. and Brodt, P. Eradication of Hepatic Metastases of Carcinoma H-59 combination chemoimmunotherapy with Liposomal Muramyl Tripeptide, 5-Fluorouracil, and Leucovorin. *Cancer Research* **52**: 6254-6257, 1992
46. Watson, S.A, Michaeli, D., Grimes, S., Morris, T., Robinson, G., Varro, A., Justin, T.A., Hardcastle, J.D. Gastrimmune raises antibodies that neutralize amidated and glycine-extended gastrin-17 and inhibit the growth of colon cancer. Cancer Res 1996; **56:** 880-885.
47. Baldwin, G. Binding of the progastrin fragments to the 78KDA gastrin-binding protein. *FEBS Lett* 1995; **359**: 97-100.
48. Makishima, R., Larkin, D., Michaeli, D., Gaginella, T.S. Active immunization against gastrin-17 with an N-terminal derived immunogen inhibits gastrin and duodenal lesions in rats. *Gastroenterol* 1995; **106**: A824.
49. Martin, F., Caignard, A., Jeannin, J.F., Leclerc, A., Martin, M. Selection of trypsin of 2 sublines of rat colon cancer cells forming progressive or regressive *tumors. Int J Cancer* 1983; **32**: 623-627.

## Claims

1. A combination suitable for use in treating a gastrin-dependent tumor, comprising:
(i) a gastrin G17-immunomimic peptide-containing immunogen, and
(ii) one or more chemotherapeutic agents for treating the tumor.

2. A combination according to Claim 1, wherein the chemotherapeutic agent is selected from 5-fluorouracil, leucovorin, levamisole, cisplatin, tumor necrosis factor and proglumide.

3. A combination according to Claim 1 or 2, wherein the gastrin G17 immunomimic peptide is conjugated to a diphtheria toxoid carrier.

4. A combination according to Claim 1 or 2, wherein the gastrin G17-immunomimic peptide-containing immunogen comprises the gastrin G17-immunomimic peptide, a protein carrier and a spacer peptide suitable for projecting the gastrin G17-immunomimic peptide away from the protein carrier and for enhancing its capacity to bind lymphocyte receptors.

5. A combination according to any of Claims 1 to 4, wherein the gastrin G17-immunomimic peptide is of SEQ ID NO: 1.

6. A combination according to any preceding Claim, further comprising a pharmaceutically acceptable carrier.

7. A combination according to any preceding Claim for treating a gastrin-dependent tumor in a patient.

8. A combination according to Claim 1 containing as chemotherapeutic agents 5-fluorouracil and leucovorin, for treating a gastrin-dependent colorectal tumor in a patient.

9. Use of a combination claimed in any one of Claims 1 to 8 in the manufacture of a formulation for use in treating a gastrin-dependent tumor.

## Patentansprüche

1. Eine Kombination, die für Benutzung bei der Behandlung eines Gastrin-abhängigen Tumors geeignet ist, welche
(i) ein Immmunogen, welches ein Gastrin G17-immunomimisches Peptid enthält, und
(ii) ein oder mehrere chemotherapeutische Mittel, um den Tumor zu behandeln,
umfaßt.

2. Eine Kombination, gemäß Anspruch 1, wobei das chemotherapeutische Mittel aus 5-Fluorouracil, Leucovorin, Levamisol, Cisplatin, Tumor-Necrosis Faktor 10 und Proglumid ausgewählt wird.

3. Eine Kombination, gemäß Anspruch 1 oder 2, wobei das Gastrin G17-immunomimische Peptid an einen Diphtherie-Toxoid Carrier konjugiert wird.

4. Eine Kombination, gemäß Anspruch 1 oder 2, wobei das Immmunogen, welches ein Gastrin G17-immunomimisches Peptid enthält, ein Gastrin G17-immunomimisches Peptid, einen Proteincarrier und ein Spacerpeptid, welches dazu geeignet ist, das Gastrin G17-immunomimische Peptid auf Abstand vom Proteincarrier zu halten und seine Fähigkeit an Lymphozytrezeptoren zu binden, verbessert, umfasst.

5. Eine Kombination, gemäß jedem der Ansprüche 1 bis 4, wobei das Gastrin G17-immunomimische Peptid SEQ ID NO: 1 ist.

6. Eine Kombination, gemäß jedem vorhergehenden Anspruch, welches weiterhin einen pharmazeutisch akzeptablen Carrier enthält.

7. Eine Kombination, gemäß jedem vorhergehenden Anspruch, um einen Gastrin-abhängigen Tumor bei einem Patienten zu behandeln.

8. Eine Kombination, gemäß Anspruch 1, welche als chemotherapeutische Mittel 5-Fluorouracil und Leucovorin enthält, um einen Gastrin-abhängigen kolorektalen Tumor bei einem Patienten zu behandeln.

9. Die Benutzung einer Kombination, gemäß jedem der Ansprüche 1 bis 8, bei der Herstellung einer Zusammensetzung zur Behandlung eines Gastrin-abhängigen Tumors

## Revendications

1. Combinaison appropriée pour une utilisation dans le traitement d'une tumeur dépendante de la gastrine, comprenant :
(i) un immunogène contenant un peptide immuno-mimétique de la gastrine G17 et
(ii) un ou plusieurs agents chimiothérapeutiques pour le traitement de la tumeur.

2. Combinaison selon la revendication 1, dans laquelle l'agent chimiothérapeutique est choisi parmi le 5-fluorouracile, la leucovorine, le lévamisole, le cis-platine, le facteur de nécrose tumorale (TNF) et le proglumide.

3. Combinaison selon la revendication 1 ou 2, dans laquelle le peptide immuno-mimétique de la gastrine G17 est conjugué à un porteur de toxoïde diphtérique.

4. Combinaison selon la revendication 1 ou 2, dans laquelle l'immunogène contenant un peptide immuno-mimétique de la gastrine G17 comprend le peptide immuno-mimétique de la gastrine G17, un véhicule de protéine et un peptide espaceur approprié pour éloigner le peptide immuno-mimétique de la gastrine G17 du véhicule de protéine et pour améliorer sa capacité à se lier aux récepteurs lymphocytes.

5. Combinaison selon l'une quelconque des revendications 1 à 4, dans laquelle le peptide immuno-mimétique de la gastrine G17 est de séquence SEQ ID n° 1.

6. Combinaison selon l'une quelconque des revendications précédentes, comprenant en outre un véhicule pharmaceutiquement acceptable.

7. Combinaison selon l'une quelconque des revendications précédentes pour le traitement d'une tumeur dépendante de la gastrine chez un patient.

8. Combinaison selon la revendication 1 contenant le 5-fluorouracile et la leucovorine en tant qu'agents chimiothérapeutiques, pour le traitement d'une tumeur colorectale dépendante de la gastrine chez un patient.

9. Utilisation d'une combinaison selon l'une quelconque des revendications 1 à 8 dans la fabrication d'une formulation pour une utilisation dans le traitement d'une tumeur dépendante de la gastrine.
